# EUROPEAN PATENT SPECIFICATION

(11) **EP 0 869 758 B1**
(45) Date of publication and mention of the grant of the patent: **28.08.2002**
(21) Application number: 96943734.2
(22) Date of filing: 11.12.1996
(51) Int. Cl.: A61F 13/15, A61F 13/56

(54) **ABSORBENT ARTICLE HAVING LATERAL BARRIERS**
SAUGFÄHIGER ARTIKEL MIT SEITENSPERREN
ARTICLE ABSORBANT A BARRIERES LATERALES

(30) Priority: 29.12.1995 US 9440 P; 27.11.1996 US 757272
(43) Date of publication of application: 14.10.1998
(73) Proprietor: KIMBERLY-CLARK WORLDWIDE, INC., Neenah, Wisconsin 54956 (US)
(72) Inventor: LARSEN, Janet, Jessie, Neenah, WI 54956 (US); DILNIK, Rebecca, Lyn, Neenah, WI 54956 (US)
(74) Representative: DIEHL GLAESER HILTL & PARTNER
(86) International application number: US9619848
(87) International publication number: WO97024093

(56) References cited:
- EP-A- 0 638 303
- WO-A-96/13238
- WO-A-96/29968
- GB-A- 2 284 767
- US-A- 4 460 364

## Description

### BACKGROUND OF THE INVENTION

The present invention relates to articles for absorbing and containing body fluids. Particularly, the invention relates to an absorbent article having lateral barriers with a predetermined amount of adhesive tack which function to occlude fluid migration from the edges of the absorbent article.

All manner and variety of disposable absorbent devices, articles and appliances have been configured for the absorption of body fluids, such as menses, urine, feces and the like are well known. Such disposable absorbent articles are expected to readily absorb any body fluid insulting the bodyfacing surface thereof, retain the fluid within the absorbent and to prevent the discharged body fluids from soiling the person and/or the adjacent clothing. Examples of such absorbent article include feminine care products, adult incontinence products, training pants and the like.

In the most basic form, the absorbent articles include several common components. Specifically, the disposable absorbent articles include a liquid-permeable, bodyfacing cover, a liquid-impermeable baffle positioned distally from the cover, and an absorbent material positioned between the cover and the baffle. The absorbent article can also include one or more layers for distributing the body fluid or layers adapted to isolate the cover from the used absorbent to give the cover a dry comfortable feel even when soiled. Typically the absorbent article has one or more means for securing the article during use. Specific types of securement can include adhesives, tapes, belts, side panels, mechanical fasteners and the like.

These absorbent devices, whether utilized as diapers, incontinence garments or sanitary napkins are subject to leakage, and in particular, leakage of liquid from the side edges of the article. Leakage from absorbent devices is generally attributed to a high concentration of fluid absorption at the point of fluid insult. This could be the result of a sudden release of body fluid onto absorbent device, overloading its absorption capability; or the result of a prolonged, steady discharge which may have caused the absorbent material in the device to become super-saturated and unable to accept, to a large degree, additional fluids from the body. Using a sanitary napkin as an illustration, menses will generally migrate radically from the point of insult and will leak from the sides. It has been suggested that at least 20-25 percent of all sanitary napkins experience side leakage. One reason for this is that, when worn, the sanitary napkin can become deformed due to dynamic forces generated as the wearer moves or alters her stationary position. Generally, the sanitary napkin deforms by bunching, twisting, and roping which are all well known in the art. The greatest deformation normally occurs within that part of the article which, in use, is located in the narrowest space between the wearer's thighs. As a result of the deformation the surface area of the sanitary napkin is greatly reduced which may lead to the soiling of the wearer's body, typically around the thigh region, and the undergarment.

A wide variety of special components and adaptations have been introduced in disposable absorbent articles in order to reduce or eliminate the incidence of side leakage. For instance, many absorbent articles include elastic structures positioned along the sides of the absorbent material and adjacent at least one of the cover or baffle. The elastic structures are intended to gather at least a portion of the side edge of the absorbent article to form walls, barriers, side seals and the like to impede the flow of liquid past the side edges of the article. In addition to leg elastic structures, absorbent articles have also included elasticized containment flaps which project from the surface of the cover in an attempt to control the movement of liquid and possibly other body wastes toward the side edges of the absorbent article.
GB-A-2284767 discloses a sanitary napkin having a liquid permeable topsheet proximate the wearer's body, a liquid impermeable backsheet distal to the wearer's body, an absorbent positioned intermediate the topsheet and the backsheet, and a pressure-sensitive bodyside adhesive secured to the bodyside surface.
EP-A-0638303 relates to a sanitary napkin comprising a liquid permeable topsheet, a liquid resistant backsheet and a liquid absorbent core sandwiched between the sheets, and elastically stretchable flaps longitudinally provided adjacent transversely opposite side edges of the napkin, and wherein said flaps have applied adhesives on the top surfaces.

A problem associated with barriers, walls and the like is that to prevent liquids from soiling the wearer, the barriers are relatively tall. During use, their size makes them uncomfortable for the wearer and the barrier may fold over and obstruct the absorbent surface. This, in turn, may contribute to the soiling the wearer instead of preventing it.

Improving the performance of a disposable absorbent article continues to be a formidable undertaking, although a number of improvements have been made in both the materials used and its construction. However, eliminating leakage, particularly along the inside of the thighs without compromising comfort and fit has not yet met the desired needs of the consumer.

Therefore, there remains a need for a sanitary napkin that will be comfortable to wear while decreasing the chance of side leakage associated with the use of sanitary napkins during the menstrual period.

### SUMMARY OF THE INVENTION

Briefly, the present invention relates to an absorbent article having longitudinal axis centrally positioned, a bodyfacing surface and a garment-facing surface. The absorbent article includes a cover disposed toward the bodyfacing surface, a baffle disposed toward the garment-facing surface and an absorbent positioned between the cover and the baffle. The absorbent article also includes a barrier positioned on either side of the central longitudinal axis for intercepting body fluids migrating toward the side edge of the absorbent article. The absorbent article further includes a pressure sensitive adhesive superposed over at least a portion of the barrier. Adhesives, in accordance with the present invention, have a rheological property, tan delta (tan δ), referenced to about 20° Centigrade of less than about 0.01 at a frequency of about 0.1 radians per second and a tan δ of less than about 0.1 at a frequency of about 1000 radians per second. In a preferred embodiment the adhesive has a tan δ which is below the line A-B. The line A-B is determined by graphically plotting frequency, in radians per second, versus tan δ at a reference temperature of about 20°C. Point A is a tan δ of less than about 0.01 at a frequency of about 0.1 radians per second and Point B is a tan δ of less than about 0.1 at a frequency of about 1000 radians per second.

In a preferred embodiment, the barrier is a foamed adhesive having the above described rheology.

The invention thus relates to an absorbent article having a central longitudinal axis, said absorbent article comprising:
a) a liquid-permeable cover (16) ;
b) a liquid-impermeable baffle (18);
c) an absorbent (20) positioned between said cover (16) and said baffle (18);
d) first and second barrier structures (26, 27) for intercepting body fluid, said barrier structures (26, 27) being positioned on opposing sides of the central longitudinal axis, said first and second barrier structures (26, 27) facilitating halting lateral flow of body fluid; and
e) a pressure sensitive adhesive (28) superposed over at least a portion of each of said first and second barrier structures (26, 27), characterised in that said adhesive (28) has a rheological property tan δ of less than about 0.01 at a frequency of about 0.1 radians per second and a tan δ of less than about 0.1 at a frequency of about 1000 radians per second.

According to one embodiment said adhesive (28) is a hot melt adhesive.

In another embodiment said adhesive (28) is secured to said cover (16) and superposed over at least 5% of said barrier structures (26, 27).

In yet another embodiment said barrier structures (26, 27) are positioned adjacent to first and second longitudinal side edges (30) of said absorbent.

According to a further embodiment said barrier structures (26, 27) extend at least 10% of the length of said longitudinal side edges (30).

In another embodiment said barrier structures (26, 27) extend substantially the length of said longitudinal side edges (30).

In yet another embodiment said adhesive (28) is foamed.

According to yet a further embodiment said barrier structures (26, 27) are hydrophilic.

In another embodiment said barrier structures (26, 27) are a foam material having a resiliency of about 15% to about 60%.

In a further embodiment said foam material has a compressibility of about 0.69 kPa (0.1 psi) to about 13.8 kPa (2 psi) at 50% compression.

In yet a further embodiment said rheological property tan δ is below a line A- B between the frequencies of about 0.1 radians per second and about 1000 radians per second wherein said line A- B is defined by graphically plotting frequency in radians per second versus tan δ of said pressure sensitive adhesive at a reference temperature of about 20C, said line A- B having as point A a tan δ of less than about 0.01 at a frequency of about 0.1 radians per second and point B a tan δ of less than about 0.1 at a frequency of about 1000 radians per second.

In a further embodiment said barrier structures (26, 27) are positioned between said cover (16) and said baffle (18).

The invention further relates to a sanitary napkin (10) having a central longitudinal axis, said napkin (10) comprising:
a) a liquid-permeable cover (16);
b) a liquid-impermeable baffle (18);
c) an absorbent (20) positioned between said cover (16) and said baffle (18);
d) first and second barrier structures (26, 27) for intercepting fluid, said barrier structures (26, 27) being secured to said cover (16) on opposing sides of the central longitudinal axis, wherein said barrier structures (26, 27) are a pressure sensitive hot melt adhesive characterized in that said adhesive has a rheological property tan δ which is below a line A- B, wherein said line A- B is defined by graphically plotting frequency in radians per second versus tan δ of said adhesive at a reference temperature of about 20°C, said line A- B having as point A a tan δ of less than about 0.01 at a frequency of about 0.1 radians per second and point B a tan δ of less than about 0.1 at a frequency of about 1000 radians per second.

In one embodiment of the sanitary napkin (10) said absorbent (20) has first and second longitudinal side edges (30) and said barrier structures (26, 27) are positioned adjacent to said longitudinal side edges (30).

In a further embodiment of the sanitary napkin (10) said barrier structures (26, 27) extend substantially the length of said longitudinal side edges (30).

In yet another embodiment of the sanitary napkin (10) said adhesive is foamed.

In a further embodiment of the sanitary napkin (10) said barrier structures (26, 27) extend above a plane of said absorbent (20).

Unexpectedly, it has been discovered that superposing an adhesive of the above qualities over at least a portion of the barrier significantly improves the effectiveness of the barrier in intercepting body fluids when compared to absorbent articles having an adhesive or a barrier alone.

The general object of the present invention is to provide an absorbent article having improved fluid containing properties. A more specific object of the invention is to provide an absorbent article having a lateral barrier with an adhesive.

Another object of the invention is to provide a sanitary napkin that is comfortable to wear and which can be readily removed with little or no pain or discomfort to the wearer.

Another object of the invention is to provide a sanitary napkin having good longitudinal liquid distribution and preventing side soiling at the edges of the article.

Other objets and advantages of the present invention will become more apparent to those skilled in the art in view of the following description and the accompanying drawings.

### BRIEF DESCRIPTION OF THE DRAWINGS

FIG. 1 is a top plan view of an absorbent article showing one embodiment of the invention.
FIG. 2 is a cross-sectional view taken along 2-2 of FIG 1.
FIG. 3 is a cross-sectional view of another embodiment of the present invention.
FIG. 4 is a graphical plot of frequency (in radians per second) versus the rheological property tan delta (tan δ) illustrating the line AB.

### DETAILED DESCRIPTION OF THE INVENTION

For ease of description, the present invention will be described as being utilized in a preferred embodiment such as a catamenial device, i.e., a sanitary napkin. However, one skilled in the art would understand that the invention is not limited thereto and will appreciate the adaptability and utility of the invention in other disposable absorbent structures such as diapers, adult incontinence articles, training pants and the like.

As used herein, the term "sanitary napkin" refers to an article which is worn by females adjacent to the pudendal region and which is intended to absorb and contain various exudates which are discharged from the body, such as, blood, menses and urine. The sanitary napkin is intended to be discarded after a single use. It is to be understood that the invention may be adapted for use in other absorbent articles such as diapers, incontinent devices and the like.

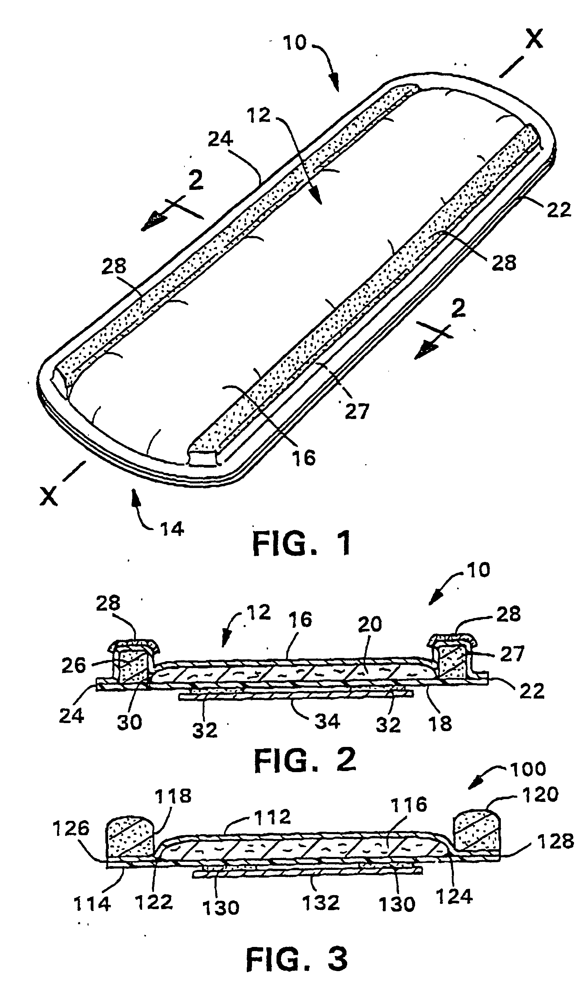

For ease of understanding when referring to the figures, similar numerals designate like parts in the different views and embodiments. Referring to Figs. 1 and 2, an embodiment of a sanitary napkin 10 is shown. Although depicted as having generally race track shape, the sanitary napkin 10 can have any variety of shapes well known to those skilled in the art. For example, hourglass, oval etc. The sanitary napkin 10 has a central longitudinal axis X--X, a bodyfacing surface 12 and a garment-facing surface 14. The term "longitudinal", as used herein, refers to a line, axis or direction in the plane of the sanitary napkin 10 that is generally aligned with or approximately parallel to a vertical plane which bisects a standing wearer into left and right body halves when the sanitary napkin 10 is worn. The bodyfacing surface 12 is generally understood as that side of the sanitary napkin 10 intended to be directed or positioned adjacent to the body of the wearer while the garment-facing surface 14 is on the opposite side and is intended to reside adjacent to the wearer's undergarments when the sanitary napkin is worn. Accordingly, when referring herein to the bodyfacing surface 12 or the garment facing surface 14 it is intended to be descriptive of a general orientation, position, or direction of the element or portion of the element.

Generally, the sanitary napkin 10 includes a cover 16, a baffle 18 and an absorbent 20 positioned between the cover 16 and the baffle 18. To prevent body fluids from reaching the sides 22 and 24 of the sanitary napkin 10 barrier structures 26 and 27 are provided. The barrier structures 26 and 27 are longitudinally positioned between the central longitudinal axis X--X and the sides 22 and 24 of the sanitary napkin 10 and preferably, are positioned parallel to the central longitudinal axis X--X. By providing longitudinal barriers 26 and 27 inward of the sides 22 and 24 the lateral migration of body fluid insulting the bodyfacing surface 12 can be directed longitudinally along the barriers 26 and 27. Thus, greater utilization of the absorbent 20 is achieved, lessening the likelihood of the sanitary napkin 10 leaking from the sides 22 and 24. The terms "disposed", "disposed on", "disposed near", "disposed at" and variations thereof are intended to mean elemental arrangement such that one element can be integral with another element, or one element can be a separate structure bonded to or placed with or placed adjacent to another element.

The sanitary napkin 10 further includes an adhesive 28 superposed over at least a portion of each barrier structure 26 and 27. Preferably, the adhesive 28 is superposed over at least about 5% of the bodyfacing surface 12 of each barrier 26 and 27. The adhesive 28 is adapted to secure only a portion of the sanitary napkin 10 to the wearer's body and preferably secures only the barriers 26 and 27 to the wearer.

It has unexpectedly been discovered that the use of an adhesive 28 in conjunction with the barriers 26 and 27 enhances the functionality of each barrier 26 and 27 in preventing liquids from leaking from the sides 22 and 24. The adhesive 28 also enhances the comfort the sanitary napkin 10 during use by retaining the barriers 26 and 27 in a proper relationship relative to the absorbent 20 and the wearer.

Looking at the sanitary napkin 10 in greater detail, the cover 16 is disposed toward the bodyfacing surface 12 of the sanitary napkin 10 and preferably is adjacent to the absorbent 20. The cover 16, which is designed to contact the wearer's body, is liquid-permeable and should retain little or no fluid in its structure so that it provides a relatively dry surface next to the wearer's skin. The cover 16 can be made from various polymeric films that are apertured to enhance fluid migration into the absorbent 20. The cover 16 can be manufactured from woven or nonwoven fibers or strands produced from natural or synthetic materials which are easily penetrated by body fluids. Thermoplastic polymer films made from polyethylene or polypropylene are preferred. Other acceptable covers might be produced by laminating film and fiber substrates. It can also be beneficial to aperture or emboss (not shown) the cover 16 to increase the rate at which the body fluids can penetrate down and into the absorbent 20.

The cover 16 can have at least a portion of its bodyfacing surface treated with a surfactant to render the cover 16 more hydrophilic. This results in permitting the insulting liquid to more readily penetrate the cover 16. The surfactant also diminishes the likelihood that the insulting fluid, such as menstrual fluids, will flow off the cover 16 rather than being absorbed by the absorbent 20.

The baffle 18 is liquid-impermeable and is disposed toward the garment-facing surface 14 of the sanitary napkin 10. Preferably, the baffle 18 will permit the passage of air and moisture vapor out of the sanitary napkin 10 while blocking the passage of body fluids. A good material is a micro-embossed, polymeric film, such as polyethylene or polypropylene, having a thickness of about 0.025 to 0.13 millimeters. Bicomponent films can also be used as well as woven and nonwoven fabrics which have been treated to render them liquid-impermeable. Another suitable material is a closed cell polyolefin foam. A closed cell polyethylene foam having a thickness ranging from about 0.5 millimeters to about 10 millimeters works well.

In a preferred embodiment of the sanitary napkin 10, the cover 16 and the baffle 18 extend beyond the absorbent 20 and are bonded together to define the longitudinal sides 22 and 24 of the sanitary napkin 10. The sides 22 and 24 enclose the absorbent 20 to prevent fluid leakage and to form a soft, comfortable side edge for the wearer. The cover 16 and baffle 18 can be bonded together using any means commonly known in the art for this purpose, such as by gluing, crimping, pressure and/or heat-sealing and ultrasonics.

The absorbent 20, which is positioned between the cover 16 and the baffle 18, is generally composed of one or more materials that are hydrophilic, compressible, conformable and non-irritating to the wearer. Acceptable materials are known in the art and include, for example, various natural or synthetic fibers, wood pulp fibers, regenerated cellulose or cotton fibers, or a blend of pulp and other fibers. The absorbent layers may also be comprised of other known materials used in absorbent articles such as cellulose sponge, hydrophilic synthetic sponge, such as polyurethane, and the like. The total absorbent capacity of the absorbent 20 should be compatible with the design exudate loading for the intended use of the sanitary napkin 10.

The absorbent 20 can contain superabsorbent materials which are effective in retaining body fluids. Superabsorbents have the ability to absorb a large amount of fluid in relation to their own weight. Typical superabsorbents used in absorbent articles, such as sanitary napkins, can absorb anywhere from 5 to 60 times their weight in body fluids. Superabsorbents can be incorporated into the absorbent 20 as separate layers or admixed with the cellulose fluff. Superabsorbents may be in the form of flakes, granules, films, particles, fibers or the like.

The barrier structures 26 and 27 are identical and therefore only one will be described. The barrier 26 is positioned laterally to the central longitudinal axis X--X, between the central longitudinal axis X--X and the side 22 of the sanitary napkin 10. The barrier 26 can reside on top of the cover 16 or desirably, is positioned between the cover 16 and the baffle 18. In a preferred embodiment, the barrier 26 is positioned between the cover 16 and the baffle 18, and adjacent to an edged 30 of the absorbent 20. This arrangement facilitates halting the lateral flow of absorbed liquids toward the side 22 through the absorbent 20 as well as unabsorbed liquids across the cover 16. The barrier 26 may have a linear shape of a line or may be curved while remaining in the area between the central longitudinal axis X--X and the side 22. Desirably, the barrier 26 is adjacent to the edge 30 following the contour of the absorbent 20. The barrier 26 extends at least 10% of the absorbent length and preferably, it extends substantially over the full length of the absorbent 20.

The barrier 26 can be hydrophilic but preferably is hydrophobic. The barrier 26 desirably is constructed of a flexible, easily compressed, resilient material. Suitable materials for forming the barrier 26 include hydrophobic polymer foams, such as, for example, polyurethane foams. Other flexible, resilient materials may be used such as foamed styrene butadiene, foamed polyethylene, foamed silicones, foamed vinyl plastics, soft sponge rubber and the like. Such foams can be obtained from Woodbridge Foam Fabricating, Inc., located at 1120 Judd Road, Chattanooga, Tennessee or from the E. N. Murry Company, Inc., having offices in Denver, Colorado.

It is important for comfort and functionality that the barrier 26 be resilient and compressible. The barrier 26 should have a resiliency in the range of about 15% to about 35%, preferably, from about 15% to about 50% and more preferably, from about 15% to about 60%. Resiliency is determined by the ASTM Test Method D3574-91, procedure H.

Compressibility can be in the range of about 0.69 kPa (0.1 psi) to about 13.8 kPa (2 psi) at 50% compression, preferably from about 2.07 kPa (0.3 psi) to about 11.73 kPa (1.7 psi) and most preferably from about 3.45 kPa (0.5 psi) to about 10.35 kPa (1.5 psi). Compressibility is determined by the ASTM Test Method D3574-91 procedure C. Desirably, the foamed polymeric material will have a density of about 0.02 grams per cubic centimeter (cm³) to about 0.1 grams per cm³.

The barrier 26 can have a width ranging from about 3 millimeters to about 12 millimeters and preferably the width is from about 3 millimeters to about 8 millimeters. The barrier 26 can have a height ranging from about 2 millimeters to about 25 millimeters; preferably, the height is from about 6 millimeters to about 15 millimeters; and most preferably, the barrier 26 has a height extending above the plane of the absorbent bodyfacing surface.

The adhesive 28 is superposed over at least a portion of the barrier 26 and preferably is secured to the cover 16 adjacent to the barrier 26. The adhesive 28 can be superposed over approximately 5% to 100% of the barrier 26 bodyfacing surface. The adhesive 28 can be configured in a substantially regular pattern or an asymmetrical pattern. For example, the adhesive 28 can be dots, ovals, swirls, various linear or non-linear arrays of adhesive longitudinally and/or transversely oriented and reticulated webs having unobstructed interstices between the adhesive fibers or combinations thereof. The adhesive 28 can have a thickness of about 0.01 millimeters to about 2 millimeters. The amount of adhesive 28 applied to the barrier 26 should be enough only to obtain sufficient adherence of the barrier 26 to the wearer to achieve the desired result and provide a satisfactory removal comfort.

The adhesive 28 can be applied to the cover 16 by techniques known in the art. For example, screen printing or extruding the adhesive 28 from one or more nozzles onto the cover 16 as described in the commonly assigned U.S. Patent No. 4,995,333 issued to Keller et at. on February 26, 1991.

The adhesive 28 deposited in accordance with the present invention may be any pressure sensitive adhesive, and preferably a hot melt adhesive, that is characterized as having specific rheological properties described below. The rheological analysis of an adhesive is a method of determining the viscoelastic property of polymers. Further explanations of polymer rheology and their measurement are discussed in: Viscoelastic Properties of Polymers, John D. Ferry, John Wiley & Sons, third edition, pages 264-280 (1980); "Studies of Triblock Copolymer-Tackifying Resin Interactions by Viscoelasticity and Adhesive Performance", Mun Fu Tse, Journal of Adhesion Science Technology, Vol 3. No. 7, pages 551-570 (1989); and test procedure ASTM-D 4440-84. It is critical to the present invention that the adhesive have a rheology value for tan δ less than about 0.01 at a frequency of about 0.1 radians per second and a tan δ value of less than about 0.1 at a frequency of about 1000 radians per second. It is to be understood that all values for tan δ herein are referenced to 20° Centigrade.

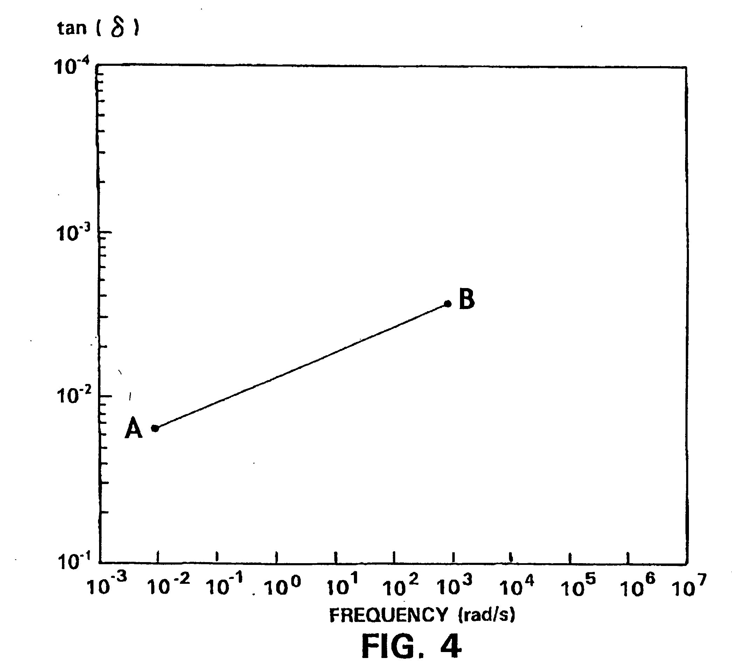

Referring to Fig. 4, preferred adhesives have a tan δ below the line A-B. Line A--B is determined by graphically plotting, on a log/log scale, frequency versus tan δ where point A is a tan δ of 0.01 at a frequency of about 0.1 radians per second and point B is a tan δ of 0.1 at a frequency of about 1000 radians per second. Adhesives having tan δ values below line AB at frequencies between about 0.1 to about 1000 radians per second lack sufficient adhesion to keep the sanitary napkin 10 securely and comfortably attached to the body of the wearer during use. However, it has been unexpectedly discovered that adhesives having this rheology property will permit the barriers 26 and 27 to remain comfortably secured to the wearer thereby enhancing the gasketing and intercepting characteristics of the barriers 26 and 27.

Suitable adhesives include, for example, Fuller 1419X and Fuller 1430 available from Fuller Adhesive located at 3530 Lexington Ave., St. Paul MN, 55126-8076.

The rheological quantities for tan δ is measured on bulk adhesive samples not suspended on any substrate and having a thickness of approximately 2 to 3 millimeters. The adhesive is cut into a 25 millimeter diameter circle and placed between two 25 millimeter parallel plate fixtures of the Rheometrics Dynamic Spectrometer, which can be obtained from Rheometrics, Inc. located at 1 PossumTown Road, Piscataway, New Jersey 08854. Adhesive samples should allowed to equilibrate at a selected test temperature before analyzing. A minicomputer governs the application of a 1% peak-to-peak shear strain to the sample. The frequency can be controlled to a fraction of a radian/sec. The values for tan δ are calculated from geometry factors, peak-to-peak amplitude of the torque signal, and phase lag of the torque output wave. Typically, a computer using RHIO's software available from Rheometrics, Inc., is used to control the operation of the apparatus. Values for time-temperature superposition are calculated between the frequencies of about 0.001 and 10⁷ radians per second using techniques known to those skilled in the adhesive art. Frequency sweeps from 0.1 rad/s to 100 < rad/s are run at 10° increments from -60°C to 120°C. The RHIO's software shifts the curves relative to a reference temperature of 20°C. From these shifted curves, a "master" curve can be generated.

Referring to Fig. 3, another embodiment of the invention is illustrated as a sanitary napkin 100. The sanitary napkin 100 has a cover 112, a baffle 114, an absorbent 116 and barrier structures 118 and 120. The cover 112 and baffle 114 extend beyond longitudinal side edges 122 and 124 of the absorbent 116 and are sealed together to form sides 126 and 128 of the sanitary napkin 100. The barrier structures 118 and 120 are secured to the cover 112 between the center longitudinal axis X--X (as seen in Fig. 1) and the sides 126 and 128 of the sanitary napkin 100. Preferably, the barrier structures 118 and 120 are secured to the cover 112 adjacent to the longitudinal side edges 122 and 124.

The barrier structures 118 and 120 are similar to those described above except they are formed by foaming an adhesive to the desired dimensions. Adhesive foaming is generally known and uses apparatus available from Nordson Corporation located at 11475 Lakefield Drive, Duluth, Georgia 30136. Suitable adhesives for foaming include, for example, Findley H5173 and Findley 995-373 which available from Findley Adhesives located at Watertown Plank Road, Wauwatosa WI, 53226.

Referring to FIGS. 2 and 3, during use the sanitary napkins 10 and 100 can be held in place by any support means or attachment means well-known for such purposes such as a garment adhesive 32 and 130. The garment adhesive 32 and 130 provides a means for securing the sanitary napkin 10 and 100 to the crotch portion of the panty. Thus, a portion or all of the garment facing surface 14 of the backsheet 26 can be coated with the garment adhesive 32 and 130. Any garment adhesive or glue used the art for such purposes can be used for the garment adhesive herein, with pressure sensitive adhesives being preferred.

The garment adhesive 32 and 130 is typically covered with a removable release liner 34 and 132 in order to keep the adhesive 32 and 130 from drying out or adhering to a surface other than the crotch portion of the panty prior to use. Commercially available release liners commonly used for such purposes can be utilized herein. The sanitary napkin 10 and 100 of the present invention is used by removing the release liner 34 and 132 and thereafter placing the sanitary napkin 10 and 100 in a panty so that the adhesive 32 and 130 contacts the panty. The adhesive 32 and 130 maintains the sanitary napkin 10 and 100 in position within the panty during use. Referring to Fig. 2, when in use the adhesive 28 secured to the cover 16 contacts and lightly adheres to the wearer. Thus, enhancing the performance of the barriers 26 and 27 in deflecting fluids longitudinally along the length of the absorbent 20 and reducing or eliminating the occurrence of side leakage from the sanitary napkin 10.

While the invention has been described with reference to a preferred embodiment and illustrated with regard to a range of optional features, those skilled in the art will appreciate that various substitutions omissions, changes and modifications may be made without departing from the attached claims Accordingly, it is intended that the foregoing description be deemed merely exemplary of the preferred scope of the present invention and not be deemed a limitation thereof.

## Claims

1. An absorbent article having a central longitudinal axis, said absorbent article comprising:
a) a liquid-permeable cover (16);
b) a liquid-impermeable baffle (18);
c) an absorbent (20) positioned between said cover (16) and said baffle (18);
d) first and second barrier structures (26, 27) for intercepting body fluid, said barrier structures (26, 27) being positioned on opposing sides of the central longitudinal axis, said first and second barrier structures (26, 27) facilitating halting lateral flow of body fluid; and
e) a pressure sensitive adhesive (28) superposed over at least a portion of each of said first and second barrier structures (26, 27), **characterised in that** said adhesive (28) has a rheological property tan δ of less than about 0.01 at a frequency of about 0.1 radians per second and a tan δ of less than about 0.1 at a frequency of about 1000 radians per second.

2. The absorbent article of claim 1 wherein said adhesive (28) is a hot melt adhesive.

3. The absorbent article of claim 1 or 2 wherein said adhesive (28) is secured to said cover (16) and superposed over at least 5% of said barrier structures (26, 27).

4. The absorbent article of one of the previous claims wherein said barrier structures (26, 27) are positioned adjacent to first and second longitudinal side edges (30) of said absorbent.

5. The absorbent article of claim 4 wherein said barrier structures (26, 27) extend at least 10% of the length of said longitudinal side edges (30).

6. The absorbent article of claim 4 wherein said barrier structures (26, 27) extend substantially the length of said longitudinal side edges (30).

7. The absorbent article of one of the previous claims wherein said adhesive (28) is foamed.

8. The absorbent article of one of the previous claims wherein said barrier structures (26, 27) are hydrophilic.

9. The absorbent article of claim 8 wherein said barrier structures (26, 27) are a foam material having a resiliency of about 15% to about 60%.

10. The absorbent article of claim 9 wherein said foam material has a compressibility of about 0.69 kPa (0.1 psi) to about 13.8 kPa (2 psi) at 50% compression.

11. The absorbent article of one of the previous claims, wherein said rheological property tan δ is below a line A-B between the frequencies of about 0.1 radians per second and about 1000 radians per second wherein said line A- B is defined by graphically plotting frequency in radians per second versus tan δ of said pressure sensitive adhesive at a reference temperature of about 20°C, said line A- B having as point A a tan δ of less than about 0.01 at a frequency of about 0.1 radians per second and point B a tan δ of less than about 0.1 at a frequency of about 1000 radians per second.

12. The absorbent article of claim 11 wherein said barrier structures (26, 27) are positioned between said cover (16) and said baffle (18).

13. A sanitary napkin (10) having a central longitudinal axis, said napkin (10) comprising:
a) a liquid-permeable cover (16);
b) a liquid-impermeable baffle (18);
c) an absorbent (20) positioned between said cover (16) and said baffle (18) ;
d) first and second barrier structures (26, 27) for intercepting fluid, said barrier structures (26, 27) being secured to said cover (16) on opposing sides of the central longitudinal axis, wherein said barrier structures (26, 27) are a pressure sensitive hot melt adhesive **characterized in that** said adhesive has a rheological property tan δ which is below a line A- B, wherein said line A- B is defined by graphically plotting frequency in radians per second versus tan δ of said adhesive at a reference temperature of about 20°C, said line A- B having as point A a tan δ of less than about 0.01 at a frequency of about 0.1 radians per second and point B a tan δ of less than about 0.1 at a frequency of about 1000 radians per second.

14. The sanitary napkin (10) of claim 13 wherein said absorbent (20) has first and second longitudinal side edges (30) and said barrier structures (26, 27) are positioned adjacent to said longitudinal side edges (30).

15. The sanitary napkin (10) of claim 14 wherein said barrier structures (26, 27) extend substantially the length of said longitudinal side edges (30).

16. The sanitary napkin (10) of one of claims 13 to 15 wherein said adhesive is foamed.

17. The sanitary napkin (10) of one of claims 13 to 16 wherein said barrier structures (26, 27) extend above a plane of said absorbent (20).

## Patentansprüche

1. Absorbierender Artikel mit einer zentralen Längsachse, wobei der absorbierende Artikel umfasst:
a) eine flüssigkeitsdurchlässige Abdeckung (16);
b) eine flüssigkeitsundurchlässige Hemmschicht (18);
c) ein Absorbens (20), das zwischen der Abdeckung (16) und der Hemmschicht (18) angeordnet ist;
d) ersten und zweiten Sperrstrukturen (26, 27) zum Auffangen von Körperfluid, wobei die Sperrstrukturen (26, 27) auf gegenüberliegenden Seiten der zentralen Längsachse angeordnet sind, wobei die ersten und zweiten Sperrstrukturen (26, 27) das Aufhalten des seitlichen Flusses von Körperfluid erleichtern; und
e) ein druckempfindliches Klebemittel (28), welches über wenigstens einem Bereich jeder der ersten und zweiten Sperrstrukturen (26, 27) zwischengelegt ist, **dadurch gekennzeichnet, dass** das Klebemittel (28) eine rheologische Eigenschaft tan δ von weniger als etwa 0,01 bei einer Frequenz von etwa 0,1 Radiant pro Sekunde und einen tan δ von weniger als etwa 0,1 bei einer Frequenz von etwa 1000 Radiant pro Sekunde aufweist.

2. Absorbierender Artikel gemäß Anspruch 1, wobei das Klebemittel (28) ein Heißschmelzklebemittel ist.

3. Absorbierender Artikel gemäß Anspruch 1 oder 2, wobei das Klebemittel (28) an der Abdeckung (16) gesichert ist und wenigstens 5 % der Sperrstrukturen (26, 27) überlagert.

4. Absorbierender Artikel gemäß einem der vorhergehenden Ansprüche, wobei die Sperrstrukturen (26, 27) benachbart zu ersten und zweiten Längsseitenkanten (30) des Absorbens angeordnet sind.

5. Absorbierender Artikel gemäß Anspruch 4, wobei die Sperrstrukturen (26, 27) sich über wenigstens 10 % der Länge der Längsseitenkanten (30) erstrecken.

6. Absorbierender Artikel gemäß Anspruch 4, wobei die Sperrstrukturen (26, 27) sich im Wesentlichen über die Länge der Längsseitenkanten (30) erstrecken.

7. Absorbierender Artikel gemäß einem der vorhergehenden Ansprüche, wobei das Klebemittel (28) geschäumt ist.

8. Absorbierender Artikel gemäß einem der vorhergehenden Ansprüche, wobei die Sperrstrukturen (26, 27) hydrophil sind.

9. Absorbierender Artikel gemäß Anspruch 8, wobei die Sperrstrukturen (26, 27) ein Schaummaterial mit einer Elastizität von etwa 15 % bis etwa 60 % sind.

10. Absorbierender Artikel gemäß Anspruch 9, wobei das Schaummaterial eine Komprimierbarkeit von etwa 0,69 kPa (0,1 psi) bis etwa 13,8 kPa (2 psi) bei 50 % Kompression aufweist.

11. Absorbierender Artikel gemäß einem der vorhergehenden Ansprüche, wobei die rheologische Eigenschaft tan δ unter einer A-B-Linie zwischen den Frequenzen von etwa 0,1 Radiant pro Sekunde und etwa 1000 Radiant pro Sekunde liegt, wobei die A-B-Linie durch graphische Darstellung einer Frequenz in Radiant pro Sekunde versus tan δ des druckempfindlichen Klebemittels bei einer Referenztemperatur von etwa 20°C definiert ist, wobei die A-B-Linie als Punkt A einen tan δ von weniger als etwa 0,01 bei einer Frequenz von etwa 0,1 Radiant pro Sekunde und als Punkt B einen tan δ von weniger als etwa 0,1 bei einer Frequenz von etwa 1000 Radiant pro Sekunde aufweist.

12. Absorbierender Artikel gemäß Anspruch 11, wobei die Sperrstrukturen (26, 27) zwischen der Abdeckung (16) und der Hemmschicht (18) angeordnet sind.

13. Hygienebinde (10) mit einer zentralen Längsachse, wobei die Binde (10) umfasst:
a) eine flüssigkeitsdurchlässige Abdeckung (16);
b) eine flüssigkeitsundurchlässige Hemmschicht (18);
c) ein Absorbens (20), das zwischen der Abdeckung (16) und der Hemmschicht (18) angeordnet ist;
d) erste und zweite Sperrstrukturen (26, 27) zum Auffangen von Körperfluid, wobei die Sperrstrukturen (26, 27) an der Abdeckung (16) auf gegenüberliegenden Seiten der zentralen Längsachse angeordnet sind, wobei die Sperrstrukturen (26, 27) ein druckempfindliches Heißschmelzklebemittel (28) sind, **dadurch gekennzeichnet, dass** das Klebemittel (28) eine rheologische Eigenschaft tan δ aufweist, die unter einer A-B-Linie liegt, wobei die A-B-Linie durch graphische Darstellung einer Frequenz in Radiant pro Sekunde versus tan δ des Klebemittels bei einer Referenztemperatur von etwa 20°C definiert ist, wobei die A-B-Linie als Punkt A einen tan δ von weniger als etwa 0,1 bei einer Frequenz von etwa 0,1 Radiant pro Sekunde und als Punkt B einen tan δ von weniger als etwa 0,1 bei einer Frequenz von etwa 1000 Radiant pro Sekunde definiert ist.

14. Hygienebinde (10) gemäß Anspruch 13, wobei das Absorbens (20) erste und zweite Längsseitenkanten (30) aufweist und die Sperrstrukturen (26, 27) benachbart zu den Längsseitenkanten (30) angeordnet sind.

15. Hygienebinde (10) gemäß Anspruch 14, wobei die Sperrstrukturen (26, 27) sich im Wesentlichen über die Länge der Längsseitenkanten (30) erstrecken.

16. Hygienebinde (10) gemäß einem der Ansprüche 13 bis 15, wobei das Klebemittel geschäumt ist.

17. Hygienebinde (10) gemäß einem der Ansprüche 13 bis 16, wobei die Sperrstrukturen (26, 27) sich über einer Ebene des Absorbens (20) erstrecken.

## Revendications

1. Article absorbant ayant un axe central longitudinal, ledit article absorbant comprenant :
a) un enveloppe (16) perméable aux liquides ;
b) un écran (18) imperméable aux liquides ;
c) un absorbant (20) positionné entre ladite enveloppe (16) et ledit écran (18) ;
d) des première et seconde structures formant barrières (26,27) pour intercepter le fluide corporel, lesdites structures formant barrières (26,27) étant positionnées sur les côtés opposés de l'axe central longitudinal, lesdites première et seconde structures formant barrières (26,27) facilitant l'arrêt de l'écoulement latéral du fluide corporel ; et
e) un adhésif (28) sensible à la pression superposé à au moins une portion de chacune desdites première et seconde structures formant barrières (26,27), **caractérisé en ce que** ledit adhésif (28) a une propriété rhéologique tan δ inférieure à environ 0,01 à une fréquence d'environ 0,1 radian par seconde et une tan δ inférieure à environ 0,1 à une fréquence d'environ 1000 radians par seconde.

2. Article absorbant selon la revendication 1, dans lequel ledit adhésif (28) est un adhésif de fusion.

3. Article absorbant selon la revendication 1 ou 2, dans lequel ledit adhésif (28) est fixé à ladite enveloppe (16) et superposé à au moins 5 % desdites structures formant barrières (26,27).

4. Article absorbant selon l'une des revendications précédentes, dans lequel lesdites structures formant barrières (26,27) sont positionnées adjacentes au premier et second bords latéraux longitudinaux (30) dudit absorbant.

5. Article absorbant selon la revendication 4, dans lequel lesdites structures formant barrières (26,27) s'étendent sur au moins 10 % de la longueur desdits bords latéraux longitudinaux (30).

6. Article absorbant selon la revendication 4, dans lequel lesdites structures formant barrières (26,27) s'étendent sensiblement sur la longueur desdits bords latéraux longitudinaux (30).

7. Article absorbant selon l'une des revendications précédentes, dans lequel ledit adhésif (28) est alvéolé.

8. Article absorbant selon l'une des revendications précédentes, dans lequel lesdites structures formant barrières (26,27) sont hydrophiles.

9. Article absorbant selon la revendication 8, dans lequel lesdites structures formant barrières (26,27) sont un matériau en mousse ayant une résilience d'environ 15 % à environ 60 %.

10. Article absorbant selon la revendication 9, dans lequel ledit matériau en mousse a une compressibilité d'environ 0,69 kPa (0,1 livre/pouce²) à environ 13,8 kPa (2 livres/pouce²) sous une compression de 50 %.

11. Article absorbant selon l'une des revendications précédentes, dans lequel ladite propriété rhéologique tan δ est inférieure à une ligne A-B entre les fréquences d'environ 0,1 radian par seconde et d'environ 1000 radians par seconde, ladite ligne A-B étant définie en traçant graphiquement la fréquence en radians par seconde en fonction de tan δ dudit adhésif sensible à la pression à une température de référence d'environ 20°C, ladite ligne A-B ayant comme point A une tan δ inférieure à environ 0,01 à une fréquence d'environ 0,1 radian par seconde et comme point B une tan δ inférieure à environ 0,1 à une fréquence d'environ 1000 radians par seconde.

12. Article absorbant selon la revendication 11, dans lequel lesdites structures formant barrières (26,27) sont positionnées entre ladite enveloppe (16) et ledit écran (18).

13. Serviette hygiénique (10) ayant un axe central longitudinal, ladite serviette (10) comprenant :
a) une enveloppe (16) perméable aux liquides ;
b) un écran (18) imperméable aux liquides ;
c) un absorbant (20) positionné entre ladite enveloppe (16) et ledit écran (18) ;
d) des première et seconde structures formant barrières (26,27) pour intercepter le fluide, lesdites structures formant barrières (26,27) étant fixées à ladite enveloppe (16) sur les côtés opposés de l'axe central longitudinal, lesdites structures formant barrières (26,27) étant un adhésif de fusion sensible à la pression, **caractérisé en ce que** ledit adhésif a une propriété rhéologique tan δ qui est inférieure à une ligne A-B, ladite ligne A-B étant définie en traçant graphiquement la fréquence en radians par seconde en fonction de tan δ dudit adhésif à une température de référence d'environ 20°C, ladite ligne A-B ayant comme point A une tan δ inférieure à environ 0,01 à une fréquence d'environ 0,1 radian par seconde et comme point B une tan δ inférieure à environ 0,1 à une fréquence d'environ 1000 radians par seconde.

14. Serviette hygiénique (10) selon la revendication 13, dans laquelle ledit absorbant (20) a des premier et second bords latéraux longitudinaux (30) et lesdites structures formant barrières (26,27) sont positionnées adjacentes auxdits bords latéraux longitudinaux (30).

15. Serviette hygiénique (10) selon la revendication 14, dans laquelle lesdites structures formant barrières (26,27) s'étendent sensiblement le long desdits bords latéraux longitudinaux (30).

16. Serviette hygiénique (10) selon l'une des revendications 13 à 15, dans laquelle ledit adhésif est alvéolé.

17. Serviette hygiénique (10) selon l'une des revendications 13 à 16, dans laquelle lesdites structures formant barrières (26,27) s'étendent au-dessus d'un plan dudit absorbant (20).
